# EUROPEAN PATENT SPECIFICATION

(11) **EP 4 469 007 B1**
(45) Date of publication and mention of the grant of the patent: **29.04.2026**
(21) Application number: 23701589.6
(22) Date of filing: 11.01.2023
(51) Int. Cl.: A61F 2/30, A61F 2/38

(54) **SURGICAL IMPLANT**
CHIRURGISCHES IMPLANTAT
IMPLANT CHIRURGICAL

(30) Priority: 28.01.2022 GB 202201116
(43) Date of publication of application: 04.12.2024
(73) Proprietor: Invibio Knees Limited, Thornton Cleveleys, Lancashire FY5 4QD (GB)
(72) Inventor: GAZE, Harry, Thornton Cleveleys, Lancashire FY5 4QD (GB)
(74) Representative: Thompson, Nicola Ruth
(86) International application number: PCT/GB2023/050046
(87) International publication number: WO 2023/144507

(56) References cited:
- EP-A1- 2 420 208
- EP-A1- 3 400 912
- US-A1- 2013 090 733
- US-A1- 2014 277 461

## Description

### TECHNICAL FIELD

The present invention relates to a surgical implant, and further relates to a method of manufacturing a surgical implant. Certain examples of the present invention relate to a knee implant, for example, a femoral implant.

### BACKGROUND

Prosthetic implants can be used to partially or completely replace diseased and/or damaged joint tissue. EP 3 400 912 A1 describes a prosthetic knee a prosthetic femoral component including a lateral condyle, a medial condyle, and a bone-contacting surface that may be formed of porous metal. US 2014/0277461 A1 describes a three-dimensional scaffold for a medical implant that includes a plurality of layers bonded to each other. US 2013/0090733 A1 describes an intervertebral spacer having an upper layer, a lower layer and a compliant layer disposed between the lower layer and the upper layer. EP 2420208 A1 relates to prosthetic intervertebral discs which include upper and lower endplates separated by a compressible core member. Prosthetic implants may include articulating surfaces that replace the bone's natural articulating surfaces. For example, an implant for a knee replacement may include a femoral implant and/or a tibial implant. A femoral implant may be implanted on the distal end of the femur and may replace the articulating surfaces of the femur. A tibial implant may be implanted on the proximal end of the tibia and may replace the articulating surfaces of the tibia. In operation, the articulating surfaces of the femoral implant articulate against the articulating surfaces of the tibial implant.

Various materials have been used for femoral and tibial implants. For example, the implants may be made from metal, for instance, cobalt-chrome. Metal implants may have a significantly higher stiffness and tensile strength than bone. As a result, metal implants can have an increased tendency to shield the underlying bone from stresses that would normally be applied to the joint during use. According to Wolff's law, bone remodels in response to applied loads. If a bone is shielded from these loads, the bone will not be exposed to the stimulus required to maintain bone mass. This can lead to a loss of bone mass that may increase the chances of the implant coming loose. In recent years, there has been increasing interest in knee implants formed from polymeric compositions that may be formulated to have mechanical properties that are more compatible with the mechanical properties of bone.

To implant a femoral implant onto the distal femoral bone, the bone may need to be resected. The implant may be held in position using a bone cement, for instance polymethylmethacrylate (PMMA). This type of fixation requires a mechanical bond to be formed between the cement and the bone, as well as a mechanical bond between the cement and the implant. In some instances, large loads transferred through the implant can cause one, or both, of these mechanical bonds to degrade, particularly over time. Furthermore, some patients may have adverse reactions to the bone cement. These factors have lead to the development of cementless implants.

Primary fixation of cementless implants may be obtained by the formation of compressive forces and subsequent shearing forces at the bone-implant interface during implantation. These compressive and shearing forces can be influenced by, for example, the interference fit and the frictional properties of the implant surface. The interference fit refers to the dimensional difference between the prepared bone cuts and the implant. The femoral bone may be cut slightly larger than the internal implant dimensions, resulting in a press fixation after implantation. The coefficient of friction may be altered by the roughness of the implant surface. In combination with the press-fit compressive forces, the roughness of the interface affects the resistance to shear forces at the implant-bone interface and can play an important role in the primary fixation of the implant. The roughness of the interface may also provide a textured surface that permits bone in-growth into the implant (secondary fixation).

It is an aim of certain examples of the present invention to solve, mitigate or obviate, at least partly, at least one of the problems and/or disadvantages associated with the prior art. In particular, it is an aim of certain examples of the present invention to provide a surgical implant and corresponding manufacturing method that results in surgical implants having more accurately determined dimensions (that is, a lower degree of variation between implants).

### BRIEF SUMMARY OF THE INVENTION

According to a first aspect of the present invention there is provided a surgical implant as defined in claim 1.

The bone interface layer provides additional stiffness to the implant body that serves both to improve fixation of the implant to resected bone, and also to resist deformation of the implant body as the implant cools following injection moulding. Particularly, and as described in greater detail below, the present inventors have identified that where dissimilar materials are used to form the implant body and the bone interface layer different degrees of thermal expansion and contraction may cause deformation as one part contracts under cooling more than the other. By forming the bone interface layer from a material including a polymer barrier layer at least 0.5 mm thick, the bone interface layer has sufficient stiffness to resist wholly or in part the forces applied to it from the contraction of the overlying polymer. This stiffness results in implants having a lower tolerance in their critical dimensions.

The bone interface layer may be at least 1.7 mm thick across a majority of its surface area. The bone interface layer may be at least 15% as thick as the thickness of the underlying implant body along an axis perpendicular to the bone interface layer across a majority of its surface area. The present inventors have identified that as well as or in place of the minimum thickness of the bone interface layer polymer barrier layer noted above, a bone interface layer specified in this way has sufficient stiffness to resist deformation under cooling.

The bone interface layer has a bend stiffness of at least 500 N/mm across a majority of its surface area. The bone interface layer has a flexural modulus of at least 1.0 GPa across a majority of its surface area. The bone interface layer has a compressive modulus of at least 4 GPa across a majority of its surface area. Thus, the bone interface layer may be characterised to give the necessary degree of stiffness as well as or in place of the dimensional characteristics noted above.

The bone interface layer may further comprise a bone-facing first porous layer. Advantageously, this provides a textured surface that permits bone in-growth into the implant for secondary fixation. The first porous layer may be applied over the whole or part of the polymer barrier layer and may extend beyond the polymer barrier layer in at least one region.

The bone interface layer may further comprise a second porous layer facing the implant body. This may serve to better couple the bone interface layer to the polymer implant body by permitting the polymer to enter into pores within the second porous layer during injection moulding. The second porous layer may be applied over the whole or part of the polymer barrier layer and may extend beyond the polymer barrier layer in at least one region. Where first and second porous layers are provided they may cover substantially the same or different regions of the polymer barrier layer. The polymer barrier layer may, in some examples, be solid: that is, it may be impermeable to gases and liquids. In some cases it may thus be referred to as a solid layer. The polymer barrier layer serves to prevent liquid polymer material passing through during an injection moulding process. That is, during moulding the polymer may passes into pores or interstitial spaces within the second porous layer but may not pass through the polymer barrier layer. In some examples the polymer barrier layer may include vent holes such that as the polymer material flows into the second porous layers air may escape to prevent any air pockets forming in the second porous layer or at least reduce the likelihood or number of air pockets. The vents will be sized to prevent the polymer passing through. For instance, the vents may have a diameter of approximately 0.05 mm. For instance, the polymer barrier layer may be a generally continuous, solid layer, with a regular or irregular array of vents. Where the second porous layer is formed from a mesh, the vents may suitably be aligned with some or all of the holes in the mesh. Accordingly, where there are first and second porous layers, polymer may be embedded into the second porous layer but will not pass through the polymer barrier layer and enter the first porous layer. In some cases the second porous layers may comprise a mesh applied to part or the whole of the polymer barrier layer. The mesh edges may be rounded to aid the polymer flowing into and through the mesh during a moulding process. In some cases the second porous layer may comprise two layers of mesh that are offset from one another so that the holes in the mesh are not aligned with one another but are fluidly connected (for instance by the holes being larger than the walls of the mesh).

The polymer barrier layer may be at least 25% of the thickness of the bone interface layer across a majority of its surface area. That is, the polymer barrier layer may be at least 25% of the total thickness of the bone interface layer when one or more porous layer (or alternative structural layers) are provided.

At least one porous layer comprises a perforated sheet, a mesh, a 3D printed layer or a layer of sintered material. Further methods of forming porous layers will be apparent to the person skilled in the art of manufacturing surgical implants.

The bone interface layer may be formed of metal, for instance titanium. Alternatively, the bone interface layer may be formed from fibre-reinforced polyaryletherketone. The implant body polymer material may also comprise a polyaryletherketone. If both the implant body and the bone interface layer comprise fibre-reinforced polyaryletherketone then the compositions are different from one another. Polyetheretherketone (PEEK) is a suitable polyaryletherketone.

The bone interface layer may be provided over the whole of or a selected region or regions of the bone-facing surface.

The implant may comprise a knee implant, for instance a femoral knee implant.

Where The implant comprises a femoral knee implant, the bone interface layer may extend onto at least part of the bone facing surface of an anterior flange and at least one condyle of the implant body.

According to a second aspect of the present invention there is provided a method of manufacturing a surgical implant, the method comprising: providing a bone interface layer; and over moulding a polymer material to form an implant body such that the bone interface layer is provided over at least a portion of a bone-facing surface of the implant body; wherein the bone interface layer is formed of a material having a higher stiffness than the polymer material; and wherein the bone interface layer includes a polymer barrier layer at least 0.5 mm thick across a majority of the surface area of the bone interface layer, the polymer barrier layer being impermeable to the polymer material.

Suitably, the surgical implant may be formed by injection moulding. The bone interface layer may be provided in or on part of a mould tool prior to the injection of polymer. This process may be referred to as insert moulding. The inclusion of the minimum thickness polymer barrier layer for the bone interface layer provides for reduced deformation of the implant as the injection moulded implant body cools, thus giving the improved implant dimensional accuracy noted above.

The implant may comprise a femoral knee implant, the bone interface layer defining at least part of an anterior flange and at least one condyle. For such a femoral implant the method may further comprise: forming the bone interface layer such that the distance between a tip of the anterior flange and a tip of the condyle has a first value; and cooling the over moulded implant body; wherein the first value is selected such that contraction of the polymer material during cooling causes the anterior flange and the condyle to splay apart such that the distance between a tip of the anterior flange and a tip of the condyle has a second predetermined value.

As noted previously, the minimum thickness of the polymer barrier layer of the bone interface layer (or one of the alternative definitions given above) serves to resist deformation of the surgical implant due to different degrees of contraction under cooling following injection moulding. In some examples a minimal amount of deformation may remain. For the case of a femoral implant, the contracting implant body pulls on the bone interface layer causing the implant to splay open. For a bone interface layer defined as above the reduced degree of splay owing to the stiff material selected may be accurately determined and used to initially mould the implant with a reduced distance between a tip of the anterior flange and a tip of the condyle such that as the implant cools the desired dimensions are reached.

### BRIEF DESCRIPTION OF THE DRAWINGS

Examples of the invention are further described hereinafter with reference to the accompanying drawings, in which:
Figure 1 is a schematic cross-sectional view of a femoral implant 10 according to an example of the present invention;
Figure 2 illustrates cross sections of test coupons where PEEK is applied to a bone interface layer;
Figure 3 illustrates splaying of a femoral implant where a PEEK implant body is over moulded on a bone interface layer relative to a PEEK implant body without a bone interface layer;
Figure 4 is a schematic cross section for a bone interface layer according to an example of the present invention;
Figure 5 are example surfaces for a bone interface layer according to an example of the present invention;
Figure 6 is a flow chart illustrating a method of manufacturing a surgical implant according to an example of the present invention; and
Figure 7 illustrates one of the manufacturing steps of the method of figure 6.

### DETAILED DESCRIPTION

The following description relates particularly to a femoral knee implant by way of example. However, the present invention is more generally applicable to any type of surgical implant, particularly but not exclusively surgical implants used in joint replacement procedures.

A knee implant according to an example of the present invention comprises an implant body formed from a polymer material. The implant body has a bone-facing surface and an articulating interface. The articulating surface may be considered as an external interface, while the bone-facing surface may be considered as an internal interface. The articulating surface may be smooth, that is with a low coefficient of friction. In the case of a femoral implant, the articulating surface may be the interface that, during use, articulates against the articulating (bearing) interface of a tibial implant. The bone-facing surface may be the interface that is implanted to face the distal portion of the femur.

The articulating surface may comprise at least one condyle, typically a first condyle and a second condyle. The first condyle and second condyle may correspond to the lateral and medial condyles of the femoral tissue of the original knee joint. In some examples, an opening may be provided between the first condyle and second condyle. The opening may define an intercondylar notch.

The bone-facing surface may comprise a bone-engaging protrusion (for instance, a stem) that extends from the bone-facing interface. Hole(s) may be made in the distal portion of the femur to receive such a bone-engaging protrusion to help secure the femoral implant in place. In some examples, the bone-facing surface may comprise two or more bone-engaging protrusions. A stiffening bone interface layer is provided over at least a portion of the bone-facing surface of the polymer implant body.

By using a polymer material (for instance, polyaryletherketone), the implant is formed having improved mechanical compatibility with bone. Accordingly, when the implant is implanted into bone, stress-shielding may be reduced and the distribution of load improved compared, for example, to metal implants. This can provide the underlying bone with sufficient biological stimulus for maintaining bone mass and/or reducing bone loss.

It has been found that, after implantation, the implant may move relative to the underlying bone (micromotion). While excessive micromotion (for instance, greater than 150 microns) may be undesirable, smaller micromotions (for instance, 40 microns or less) may be desirable to promote bone in-growth. Accordingly, to tailor the mechanical properties of at the bone-engaging surface to optimise bone in-growth, according to the present invention a bone interface layer is provided over at least a portion of the bone-facing surface of the implant.

In certain examples of the present invention, the bone interface layer includes a porous layer facing the bone (on at least part of an internal surface). The pores in a porous layer of the bone interface layer may provide openings for bone in-growth at the implant/bone interface, facilitating fixation of the implant at the site of implantation. Such cementless fixation at the bone/implant interface may offer improved load transfer across the interface and into the for instance, femoral and/or tibial bone. According to examples of the present invention, the bone interface layer is also formed of a material having a higher stiffness than the polymer material of the implant body. This increased stiffness allows micromotions at the bone interface to be controlled to levels that improve bone-ingrowth, for instance, while reducing the risk of excessive micromotion that may lead to for instance, implant loosening. The degree of micromotion can also be controlled to favour the growth of certain cells (for instance, bone cell growth) over the growth of other cells (for instance, fibrous tissue).

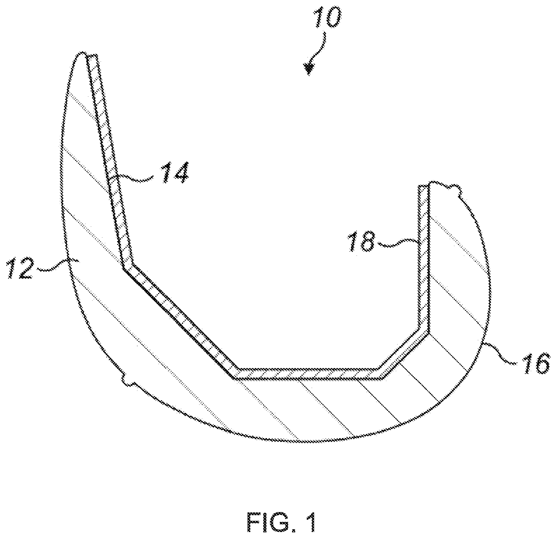

Referring to figure 1, this is a schematic cross-sectional view of a femoral implant 10 according to an example of the present invention. The femoral implant 10 comprises an implant body 12 comprising a polymer material, which may suitably comprise polyaryletherketone (for instance, PEEK). The implant body 12 comprises a bone-facing surface 14 and an articulating surface 16. A bone interface layer 18 is provided over at least a portion of the bone-facing surface 14. The bone interface layer 18 is formed of a material having a higher stiffness than the polymer material of the implant body 12. The bone interface layer 18 may include a porous material to improve bone-ingrowth. In the example of figure 1, the bone interface layer 18 is of uniform thickness and lines substantially the entire bone-facing surface 14, though this is not essential.

The bone interface layer 18 may be formed from a metal, for instance titanium. It will be appreciated that metals suitable for forming the bone interface layer 18 are stiffer than the polymer implant body 12. In some cases the bone interface layer 18 may be formed from a polymer or a reinforced polymer that is stiffer than the polymer implant body 12. More generally, any material that is biocompatible, suitably for over moulding with the implant body polymer 12, and stiffer than the implant body to provide the advantageous properties outlined above may be used to form the bone interface layer. In the following examples of the present invention a metal bone interface layer 18 is referenced, particularly titanium, though it will be appreciated that the invention is not restricted to this. Similarly, the present invention is not restricted to the polymer implant body being PEEK even though this is used in certain examples.

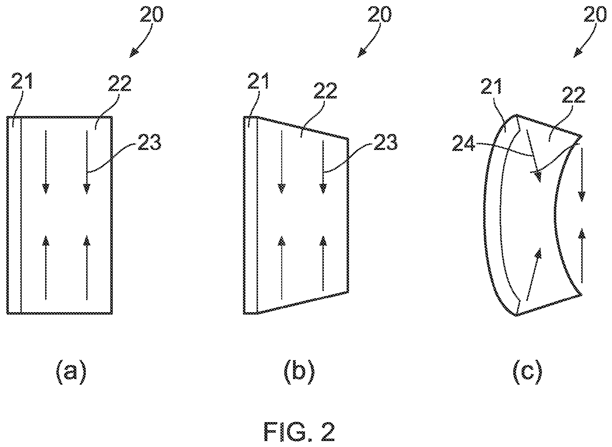

The present inventors have identified that cooling following injection moulding of the implant body 12 over the bone interface layer 18 can lead to distortion. This arises because the polymer of the implant body 12 contracts more during cooling than the bone interface layer 18 owing to their different temperature coefficients. For a PEEK implant body 12 and a titanium bone interface layer 18 this difference can be pronounced. Figure 2 schematically illustrates this distortion through a test coupon 20 in which a titanium insert 21, for instance a sample of a bone interface layer 18, is over moulded with a layer of PEEK 22. The PEEK layer 22 is relatively thick compared to the titanium insert 21. Part (a) of figure 2 shows the test coupon 20 immediately after it has been moulded and is starting to cool. As the PEEK layer 22 cools, the PEEK crystalises and tries to shrink more than the titanium insert 21, as indicated by arrows 23. Part (b) shows the test coupon 20 as cooling progresses. Contraction of the PEEK 22 on one side is constrained by the titanium insert 21, with the PEEK 22 beginning to deform as the side opposite the titanium insert can contract more. Part (c) shows the test coupon 20 once cooled to an ambient temperature. The contraction of the PEEK 22 effectively tries to shorten the titanium insert 21 as the PEEK is interlocked into pores within the surface of the titanium insert. The only way this is possible is for the titanium insert 21 to bend as illustrated, thus allowing the PEEK to shrink as indicated by arrows 24. This deformation can continue to occur for a period of time after the test coupon 20 has been ejected from the mould.

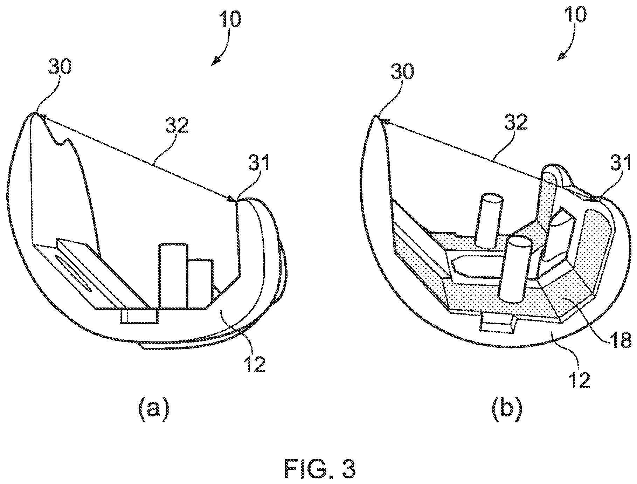

Turning to figure 3, for the example of a femoral implant 10, this distortion under cooling after injection moulding manifests as the femoral implant 10 splaying open. Part (a) of figure 3 shows a femoral implant 10 injection moulded using PEEK without a bone interface layer. As the whole implant is a single material there is no difference in temperature coefficient and so any contraction of the implant body 12 during cooling is uniform and predictable. Particularly, nominal implant geometry is maintained during cooling. That is, the shape imparted to the implant body by the injection moulding process is maintained during cooling (though a predictable degree of overall part shrinkage may occur). Accordingly, implant bodies 12 with a low variability can be achieved. A key dimension for a femoral implant 10 is the distance between the tip of a condyle 30 and a tip of an anterior flange 31. This dimension, labelled 32 in figure 3 has a low tolerance for implant bodies formed using the same mould using PEEK only.

Part (b) of figure 3 shows a femoral implant 10 also formed by injection moulding a PEEK implant body 12 using the same mould, differing from part (a) only in that a titanium bone interface layer 18 was positioned within the mould such that it lines the bone-facing surface 14 of the implant body 12. Under cooling the distortion demonstrated in figure 2 results in the condyles and the anterior flange splaying apart such that the distance 32 between the tip of a condyle 30 and a tip of an anterior flange 31 increases. This occurs because the bone interface layer 18 is formed as a single piece and the PEEK implant body 12 effectively pulls around the outside of the bends in the bone interface layer 18 as it contracts. There may be further deformation within localised parts of the bone interface layer 18 in addition to the global splaying effect. The splaying effectively opens up the distal regions of the femoral implant 10, increasing the angle between bone-facing surfaces of the condyles and the anterior flange.

It will be appreciated that this splaying open of the femoral implant 10 will reduce the tightness of the interference fit of the implant upon the resected bone. Furthermore, the splaying is an uncontrolled process in which there is significant variation between different implants manufactured using identical bone interface layers 18. Accordingly, even if the dimensions of the mould tool and the bone interface layer 18 prior to injection moulded are adjusted to take account for splaying during cooling, the resulting implants would suffer from an unacceptably high tolerance in distance 32 between the tip of a condyle 30 and a tip of an anterior flange 31. It is possible to reduce the degree of splay by cooling the implant 10 within a jig that clamps the implant 10 after it is removed from the mould tool to constrain the distance 32 and resist splaying outwards. The jig may constrain the distance 32 to the nominal distance for the implant, or to a slightly reduced distance to account for a reduced splaying action once the implant is removed from the jig. While jig cooling has been demonstrated by the inventors to reduce splaying, it has not been possible to remove the splaying effect completely nor reduce the tolerance for the distance 32 to acceptable levels. Furthermore, it has been found that for jig cooled implants, after annealing the implant body (which may be a required processing step for a surgical implant to relieve stress induced in the implant body during moulding) significant further splaying may occur, which may equal the degree of splaying for implants cooled without using a jig. Furthermore, both of these mitigation techniques require significant time and experimentation to achieve, which requires the costly adaptation of mould tools and/or jigs each time a new implant design is developed.

The present inventors have further identified that a solution to this problem of implant distortion is to change the structure of the bone interface layer in order to resist bending or distortion as an over moulded polymer body cools. By increasing the stiffness of the bone interface layer sufficiently relative to the polymer of the implant body, distortion such as splaying for a femoral implant may be reduced to levels that fall within an acceptable tolerance band for the implant. Advantageously, by directly mitigating implant distortion through changes to the bone interface layer, the time consuming and costly process of trial and error adaptation of mould tools and use of jigs may be avoided to achieve a similar reduction in tolerance limits for implants. A further advantage is that a different material or structure for the bone interface layer may substituted using the same mould tool without requiring the mould tool to be adapted.

While the increased stiffness imparted by the bone interface layers of certain examples of the present invention has the benefit of reducing post-mould distortion of over moulded titanium cementless implants, including an increased retention of moulded implant geometry, in some examples of the present invention this is at the expense of a reduction of polymer in the cross section of the implant, with a resulting reduction in the maximum polymer sheer capability. It will be appreciated that the increase in stiffness of the bone interface layer to resist deformation post moulding is to be balanced with the desirable property of a polymer implant to be flexible and transfer stress to the bone to avoid the problem of stress shielding experienced with metal implants. The stiffness of the material of the bone interface layer may be selected to optimise the desired degree of micromotion of the implant relative to the underlying bone, while minimising the occurrence of post-mould distortion. The extent of micromotion should be sufficiently low to avoid significant risks of implant loosening. However, a degree of micromotion should be retained to promote bone in-growth.

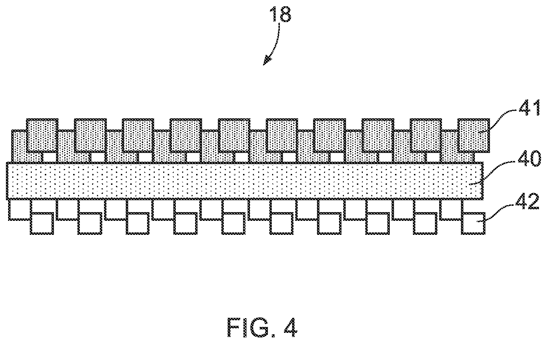

Referring now to figure 4, this illustrates a structure of a material for a bone interface layer 18 in accordance with an example of the present invention. The bone interface layer according to certain examples comprises a polymer barrier layer 40. The polymer barrier layer may, in some examples, be solid: that is, it may be impermeable to gases and liquids. In some cases it may thus be referred to as a solid layer. The inclusion of polymer barrier layer 40 serves to provide a significant degree of rigidity to the bone interface layer 18. Additionally, the polymer barrier layer 40 prevents polymer from the implant body 12 from passing through the bone interface layer 18 during injection moulding, which might otherwise occur if the full depth of the bone interface layer 18 were porous. In accordance with certain examples of the present invention, a solid Barrier layer 40 with a minimum thickness of 0.5 mm across a majority of the surface are of the bone interface layer 18 has been found to give the bone interface layer sufficient rigidity to resist significant deformation under cooling after over moulding the implant body 12. In some example, edges regions of the bone interface layer 18 may not include a polymer barrier layer 40, or it may be thinner in edge regions.

Figure 4 further shows a bone-facing first porous layer 41 that may be present in the bone interface layer 18 according to certain examples. The first porous layer 41 may allow for bone ingrowth aiding secondary fixation as described above. The first porous layer 41 may thus be referred to as a bone facing porous layer. The bone interface layer may also increase the coefficient of friction at the bone-facing interface. This may aid primary fixation of the implant. Additionally, figure 4 further shows a second porous layer 42 that may be present in the bone interface layer 18 according to certain examples. The second porous layer 42 faces the polymer implant body and serves to interlock the implant body as polymer flows into the pores. The second porous layer 42 may thus be referred to as a polymer facing porous layer. It will be appreciated that examples of the present invention may exclude one or both porous layers 41, 42. Where one or more porous layers 41, 42 are provided, along with the polymer barrier layer 40, a minimum total thickness for the bone interface layer of 1.7 mm across a majority of the surface are of the bone interface layer 18 has been found to give the bone interface layer sufficient rigidity to resist significant deformation under cooling after over moulding the implant body 12. This total thickness of the bone interface layer includes the thickness of the polymer barrier layer 40. In some examples, sufficient rigidity of the bone interface layer 18 is provided even when the polymer barrier layer 40 is less than 0.5 mm. In some examples where a polymer barrier layer 40 is included, one or more porous layer 41, 42 may extend beyond a periphery of the polymer barrier layer 40.

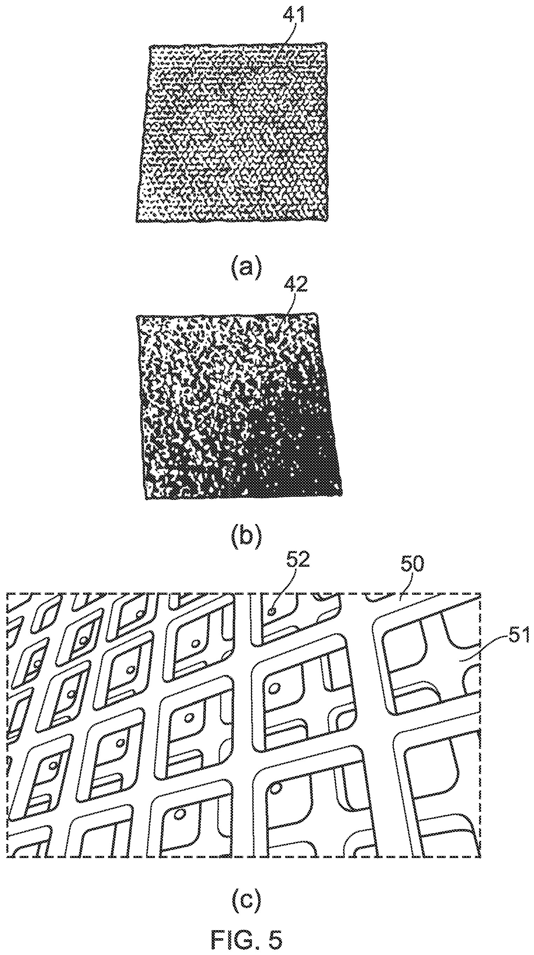

During moulding the polymer may passes into pores or interstitial spaces within the second porous layer but may not pass through the polymer barrier layer. In some examples the polymer barrier layer may include vent holes such that as the polymer material flows into the second porous layers air may escape to prevent any air pockets forming in the second porous layer or at least reduce the likelihood or number of air pockets. This is illustrated in figure 5, part (c) as discussed below. The vents will be sized to prevent the polymer passing through. For instance, the vents may have a diameter of approximately 0.05 mm. For instance, the polymer barrier layer may be a generally continuous, solid layer, with a regular or irregular array of vents.

Where the second porous layer is formed from a mesh, the vents may suitably be aligned with some or all of the holes in the mesh. Accordingly, where there are first and second porous layers, polymer may be embedded into the second porous layer but will not pass through the polymer barrier layer and enter the first porous layer. In some cases the second porous layers may comprise a mesh applied to part or the whole of the polymer barrier layer. The mesh edges may be rounded to aid the polymer flowing into and through the mesh during a moulding process. In some cases the second porous layer may comprise two layers of mesh that are offset from one another so that the holes in the mesh are not aligned with one another but are fluidly connected (for instance by the holes being larger than the walls of the mesh).

In some examples the bone interface layer 18 may not include a polymer barrier layer 40: there may only be a single porous layer or two dissimilar porous layers 41, 42. Sufficient rigidity may be provided where the thickness of the porous layer (or both in combination) exceeds 1.7 mm across a majority of the surface are of the bone interface layer 18. As will be expanded upon below, at least one of the porous layers may comprise a perforated sheet, a mesh, a 3D printed layer or a layer of sintered material. The skilled person will be familiar with such techniques for forming porous layers or surfaces as a known in the art of surgical implants.

Furthermore, where a polymer barrier layer 40 is included with one or more porous layers 41, 41, sufficient rigidity may be provided where the thickness of the polymer barrier layer 40 is at least 25% of the total thickness of the bone interface layer 18 across a majority of the surface are of the bone interface layer 18.

Turning now to figure 5, this illustrates the first and second porous layers 41, 42 of samples of the bone interface layer 18 of figure 4. Part (a) illustrates an example bone facing porous layer 41, and in the particular example illustrated this comprises sintered metal, for instance titanium, deposited on a titanium polymer barrier layer 40. Part (b) illustrates an example implant body facing porous layers 42, and in the particular example illustrated this comprises a titanium mesh layer welded or otherwise bonded to on a titanium sold layer 40. Part (c) illustrates a bone interface layer 18 according to a further example including a second porous layer 42 comprising a first and second layers of mesh 50, 51 with rounded edges, each mesh layer being offset. The polymer barrier layer 40 is visible through the mesh layers 50, 51. Vent holes 52 are visible, the vent holes 52 serving to allow air to escape during moulding, as noted above.

It has been found that sufficient rigidity to resist significant distortion can be provided when the total thickness of the bone interface layer 18 (comprising one or more of a polymer barrier layer 40 and first and second porous layers 41, 42) is at least 15% as thick as the thickness of the underlying implant body along an axis perpendicular to the bone interface layer across a majority of its surface area. That is, given that the distortion or warpage force is dependent in part upon the thickness of the polymer implant body 12 bonded to the bone interface layer 18, it is the relative proportional thickness that is a key criteria.

The examples of the present invention presented above seek to quantify the bone interface layer 18 in terms of its thickness either in absolute terms or as a percentage of the underlying polymer body 12. In the present invention it has been found that sufficient rigidity to resist significant distortion can be provided when the bone interface layer 18 has a flexural modulus of at least 1.0 GPa across a majority of its surface area or wherein the bone interface layer has a compressive modulus of at least 4 Gpa across a majority of its surface area. These quantitative measures of the flexibility of the bone interface layer 18 may be in place of or in addition to the quantitative measures given above. For instance, in some examples a bone interface layer 18 including a polymer barrier layer 40 at least 0.5 mm thick (and optionally one or more porous layer 41, 42) will necessarily have a flexural modulus of at least 1.0 Gpa and a compressive modulus of at least 4 Gpa.

According to one particular example, a bone interface layer 18 may comprise a porous scaffold made from three layers of 0.2 mm thick perforated titanium sheet (with an average pore size of 700µm) sintered together either side of a 0.5 mm thick solid sheet of titanium. The six individual bonded layers total a thickness of 1.7 mm. This example meets the flexural and compressive moduli detailed above.

The bone interface layer may comprise one or more porous meshes, optionally applied to a polymer barrier layer. The mesh and indeed the polymer barrier layer may suitably be formed of a metal. Any suitable biocompatible metal may be used. Suitable metals include stainless steel, cobalt-chrome, titanium and titanium alloy. Alternatively, the mesh and indeed the polymer barrier layer may be formed of a fibre-reinforced polyaryletherketone.

In some examples, the mesh forming one or more porous layer may comprise multiple overlying layers of mesh material. The openings of at least one layer of mesh material may be partially offset with respect to the openings of at least one other layer of mesh material to provide channels through the thickness of the mesh. The offsetting may provide the channels with a degree of tortuosity. Such channels may promote bone in-growth through the bone interface layer.

As previously noted, the extent and nature of coverage of the bone interface layer 18 over the bone-facing interface of the polymer body 12 may vary. For example, the bone interface layer may be provided over a selected region or selected regions of the bone-facing interface. Alternatively, the bone interface layer may be provided over substantially the entirety of the bone-facing interface.

It may also be possible to vary the stiffness profile of the bone interface layer or to vary the stiffness of the bone interface layer over different regions of the bone-facing interface. For example, the relative thickness and extent of coverage of each of the optional polymer barrier layer and first and second porous layers may vary. Furthermore, additional layers of material may be provided in certain regions to provide additional reinforcement. The thickness of each layer may be non-uniform.

The disposition of the bone interface layer upon the bone-facing surface of the implant may vary significantly. The bone interface layer may be applied to selected areas of the bone-facing interface. The bone interface layer may be applied discontinuously across the bone-facing surface. For instance, strips of bone interface layer may be applied at spaced intervals. This pattern, however, is merely illustrative and other discontinuous patterns may be employed.

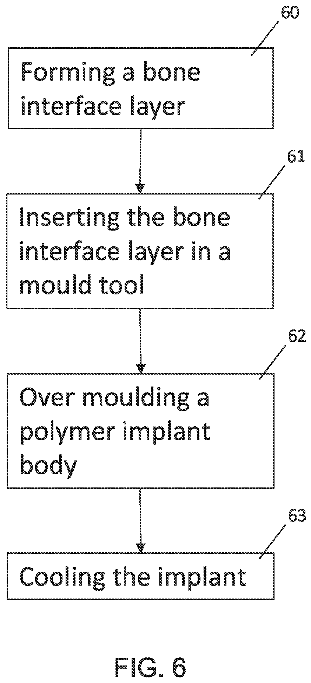

Referring now to figure 6, a method of manufacturing a surgical implant will now be described. At step 60 a bone interface layer is formed. This may comprise forming a flat sheet. In one example this may comprise welding together a solid titanium sheet sandwiched by titanium mesh layers. This sheet may then be cut to size and shape. For a femoral implant this comprises cutting a shape corresponding to at least part of the inside surfaces of the implant (corresponding to the resected surfaces of the femur).

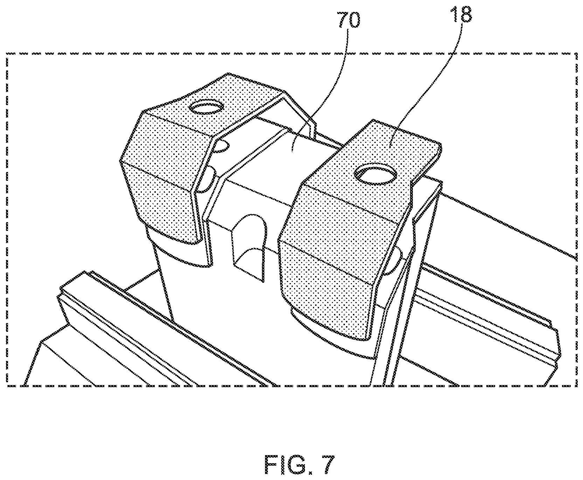

At step 61 the bone interface layer 18 is inserted into the mould tool. As illustrated in figure 7, this may comprise fitting the bone interface layer to an inner surface 70 of the mould tool. At step 62 the mould tool is closed to form the mould cavity and a polymer is injection moulded over the bone interface layer 18. In particular, the bone interface layer 18 may include a porous layer (also referred to as a polymer facing porous layer or polymer interface layer) facing into the mould cavity and the polymer is moulded over the polymer facing porous layer. As noted above, the polymer may flow into spaces within the polymer interface layer. In one example the polymer forming the implant body 12 is PEEK and the injected PEEK is heated to 390°C and injected under 1000 Bar of pressure at an injection speed for 10 m/s. At step 63 the implant is cooled. Particularly, the PEEK implant body 12 may be cooled to 190°C prior to removal from the mould tool. The cooling time inside the tool may be 100 s. The implant may further cool to the ambient temperature outside of the mould tool.

While the use of bone interface layers as described above reduces the extent of post-mould distortion, is may not remove it completely (without at least stiffening the bone interface layer to such an extent that other desirable properties of a polymer implant are lost). Accordingly, where the implant is a femoral implant, in some examples of the present invention the bone interface layer (along with the mould tool) may be formed such that the distance between a tip of the anterior flange and a tip of the condyle has a first value selected such that contraction of the polymer material during cooling causes the anterior flange and the condyle to splay apart such that the distance between a tip of the anterior flange and a tip of the condyle has a second predetermined value. That is, the expected residual splaying of post-mould distortion may be accounted for by slightly adjusting the shape of the bone interface layer and hence the moulded implant body. The skilled person will appreciate how this optimisation is extensible to other types of implant where distortion may take different forms.

As noted above, the polymer implant body may comprise polyaryletherketone. A polyaryletherketone may have repeating units of formula (I) below: 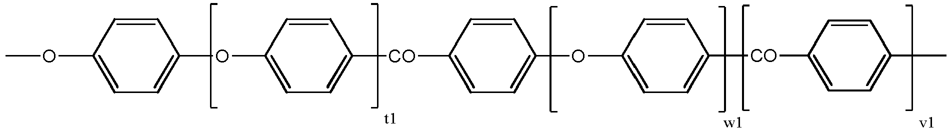 where t1 and w1 are independently represent 0 or 1 and v1 represents 0, 1 or 2.

The polyaryletherketone suitably includes at least 90, 95 or 99 mol % of repeat unit of formula I. The polyaryletherketone suitably includes at least 90, 95 or 99 weight % of repeat unit of formula I.

The polyaryletherketone may comprise or consist essentially of a repeat unit of formula I. Preferred polymeric materials comprise (or consist essentially of) a said repeat unit wherein t1=1 , v1=0 and w1=0; t1=0, v1=0 and w1=0; t1=0, w1 =1 , v1=2; or t1=0, v1=1 and w1=0. More preferably, the polyaryletherketone comprises (for instance, consists essentially of) the repeat unit I, wherein t1 =1 , v1 =0 and w1 =0; or t1=0, v1=0 and w1 =0. The most preferred polyaryletherketone comprises (especially consists essentially of) a said repeat unit wherein t1=1 , v1=0 and w1=0.

The polyaryletherketone may suitably be selected from a group including polyetheretherketone, polyetherketone, polyetherketoneetherketoneketone and polyetherketoneketone. According to examples of the present invention, the polymer is particularly polyetheretherketone (PEEK).

The polyaryletherketone may have a Notched Izod Impact Strength (specimen 80mm x 10mm x 4mm with a cut 0.25mm notch (Type A), tested at 23°C, in accordance with ISO180) of at least 4 KJm⁻² , preferably at least 5 KJm⁻², more preferably at least 6 KJm⁻². The Notched Izod Impact Strength, measured as mentioned above, may be less than 10 KJm⁻², suitably less than 8 KJm⁻². The Notched Izod Impact Strength, measured as mentioned above, may be at least 3 KJm⁻², suitably at least 4 KJm⁻², preferably at least 5 KJm⁻². The impact strength may be less than 50 KJm⁻², suitably less than 30 KJm⁻².

The polyaryletherketone may have a melt viscosity (MV) of at least 0.06 kNsm⁻², preferably has a MV of at least 0.09 kNsm⁻², more preferably at least 0.12 kNsm⁻², especially at least 0.15 kNsm⁻². Advantageously, the MV may be at least 0.35 kNsm⁻² and especially at least 0.40 kNsm⁻². An MV of 0.45 kNsm⁻² may be particularly advantageous.

Unless otherwise stated, melt viscosity (MV) is measured using a Bohlin Instruments RH2000 capillary rheometer according to ISO 1 1443 operating at 340°C and a shear rate of 1000 s⁻¹ using a 0.5 mm (capillary diameter) x 8.0 mm (capillary length) die with entry angle 180°C. Granules may be loaded into the barrel and left to pre-heat for 10 minutes. The viscosity may be measured once steady state conditions are reached and maintained, nominally 5 minutes after the start of the test. The polyaryletherketone may have a MV of less than 1.00 kNsm⁻² , preferably less than 0.5 kNsm⁻² . The polyaryletherketone may have a MV in the range 0.09 to 0.5 kNsm⁻² , preferably in the range 0.14 to 0.5 kNsm⁻² , more preferably in the range 0.4 to 0.5 kNsm⁻² .

The polyaryletherketone may have a tensile strength, measured in accordance with IS0527 (specimen type 1 b) tested at 23°C at a rate of 50mm/minute of at least 20 MPa, preferably at least 60 MPa, more preferably at least 80 MPa. The tensile strength is preferably in the range 80-1 10 MPa, more preferably in the range 80-100 MPa.

The polyaryletherketone may have a flexural strength, measured in accordance with IS0178 (80mm x 10mm x 4mm specimen, tested in three-point-bend at 23°C at a rate of 2mm/minute) of at least 50 MPa, preferably at least 100 MPa, more preferably at least 145 MPa. The flexural strength is preferably in the range 145-180MPa, more preferably in the range 145-164 MPa. The polyaryletherketone may have a flexural modulus, measured in accordance with IS0178 (80mm x 10mm x 4mm specimen, tested in three-point-bend at 23°C at a rate of 2mm/minute) of at least 1 GPa, suitably at least 2 GPa, preferably at least 3 GPa, more preferably at least 3.5 GPa. The flexural modulus is preferably in the range 3.5-4.5 GPa, more preferably in the range 3.5-4.1 GPa.

The polyaryletherketone may be amorphous or semi-crystalline. The polyaryletherketone is preferably crystallisable. The polyaryletherketone may be semi-crystalline. The level and extent of crystallinity in a polymer may be measured by wide angle X-ray diffraction (also referred to as Wide Angle X-ray Scattering or WAXS), for example as described by Blundell and Osborn (Polymer 24, 953, 1983). Alternatively, crystallinity may be assessed by Differential Scanning Calorimetry (DSC).

The level of crystallinity of said polyaryletherketone may be at least 1 %, suitably at least 3%, preferably at least 5% and more preferably at least 10%. In especially preferred embodiments, the crystallinity may be greater than 25%. It may be less than 50% or less than 40%. The main peak of the melting endotherm (Tm) of said polyaryletherketone (if crystalline) may be at least 300°C.

The polyaryletherketone described above may be used to form the implant body. For example, the polyaryletherketone described above may be used to form a polymer composition that is moulded to form the implant body. Examples of suitable moulding methods include injection moulding and compression moulding. Preferably, the implant body is formed by injection moulding.

In some examples, the polymer composition may include a contrast agent, for example, barium sulphate. For example, barium sulphate may be present in the polymer composition in an amount of 2 to 20 weight % of the total weight of the polymer composition, for instance, 3 to 10 weight %. In a preferred embodiment, the amount of barium sulphate may be 4 to 8 weight %, more preferably 4 to 6 weight %. In a most preferred embodiment, the amount of barium sulphate may be 5 weight %.

Throughout this specification, the words "comprise" and "contain" and variations of them mean "including but not limited to", and they are not intended to (and do not) exclude other components, integers or steps. Throughout this specification, the singular encompasses the plural unless the context otherwise requires. In particular, where the indefinite article is used, the specification is to be understood as contemplating plurality as well as singularity, unless the context requires otherwise. Throughout this specification, the term "about" is used to provide flexibility to a range endpoint by providing that a given value may be "a little above" or "a little below" the endpoint. The degree of flexibility of this term can be dictated by the particular variable and can be determined based on experience and the associated description herein.

Features, integers or characteristics described in conjunction with a particular aspect or example of the invention are to be understood to be applicable to any other aspect or example described herein unless incompatible therewith. All of the features disclosed in this specification, and/or all of the steps of any method or process so disclosed, may be combined in any combination, except combinations where at least some of such features and/or steps are mutually exclusive. The invention is not restricted to the details of any foregoing examples. The invention extends to any novel feature or combination of features disclosed in this specification. It will be also be appreciated that, throughout this specification, language in the general form of "X for Y" (where Y is some action, activity or step and X is some means for carrying out that action, activity or step) encompasses means X adapted or arranged specifically, but not exclusively, to do Y.

## Claims

1. A surgical implant (10) comprising:
an implant body (12) comprising a polymer material, said implant body (12) having a bone-facing surface (14); and
a bone interface layer (18) provided over at least a portion of the bone-facing surface (14);
wherein the bone interface layer (18) is formed of a material having a higher stiffness than the polymer material; and
wherein the bone interface layer (18) includes a polymer barrier layer (40) at least 0.5 mm thick across a majority of the surface area of the bone interface layer (18), the polymer barrier layer (40) being impermeable to the polymer material; **characterised in that** the bone interface layer (18) has a bend stiffness of at least 500 N/mm across a majority of its surface area;
wherein the bone interface layer (18) has a flexural modulus of at least 1.0 GPa across a majority of its surface area; or
wherein the bone interface layer (18) has a compressive modulus of at least 4 GPa across a majority of its surface area.

2. An implant (10) according to claim 1, wherein the bone interface layer (18) is at least 1.7 mm thick across a majority of its surface area; or
wherein the bone interface layer (18) is at least 15% as thick as the thickness of the underlying implant body (12) along an axis perpendicular to the bone interface layer (18) across a majority of its surface area.

3. An implant (10) according to any one of the preceding claims, wherein the bone interface layer (18) further comprises a bone-facing first porous layer (41).

4. An implant (10) according to any one of the preceding claims, wherein the bone interface layer (18) further comprises a second porous layer (42) facing the implant body (12).

5. An implant according to claim 4, wherein the polymer barrier layer includes vent holes (52) sized to prevent the polymer material passing through.

6. An implant (10) according to claim 4 or claim 5, wherein the polymer barrier layer (40) is at least 25% of the thickness of the bone interface layer (18) across a majority of its surface area.

7. An implant (10) according to any one of claims 4 to 6, wherein at least one porous layer (41, 42) comprises a perforated sheet, a mesh, a 3D printed layer or a layer of sintered material.

8. An implant (10) according to any one of the preceding claims, wherein the bone interface layer (18) is formed of metal , optionally wherein the bone interface layer (18) is formed of titanium.

9. An implant (10) according to any one of claims 1 to 7, wherein the bone interface layer (18) is formed from fibre-reinforced polyaryletherketone, optionally wherein the polyaryletherketone is polyetheretherketone, PEEK.

10. An implant (10) according to any one of the preceding claims, wherein the implant body (12) polymer material comprises a polyaryletherketone, optionally wherein the polyaryletherketone is polyetheretherketone, PEEK.

11. An implant (10) according to any one of the preceding claims, wherein the bone interface layer (18) is provided over a selected region or selected regions of the bone-facing surface (14); or
wherein the bone interface layer (18) is provided over substantially the entirety of the bone-facing surface (14).

12. An implant (10) according to any one of the preceding claims, wherein the implant (10) comprises a knee implant.

13. An implant (10) according to claim 12, wherein the knee implant comprises a femoral knee implant, optionally wherein the bone interface layer (18) extends onto at least part of the bone facing surface of an anterior flange and at least one condyle of the implant body (12).

14. A method of manufacturing a surgical implant (10), the method comprising:
providing a bone interface layer (18); and
over moulding a polymer material to form an implant body (12) such that the bone interface layer (18) is provided over at least a portion of a bone-facing surface (14) of the implant body (12);
wherein the bone interface layer (18) is formed of a material having a higher stiffness than the polymer material; and
wherein the bone interface layer (18) includes a polymer barrier layer (40) at least 0.5 mm thick across a majority of the surface area of the bone interface layer (18), the polymer barrier layer (40) being impermeable to the polymer material; and
wherein the bone interface layer (18) has a bend stiffness of at least 500 N/mm across a majority of its surface area;
wherein the bone interface layer (18) has a flexural modulus of at least 1.0 GPa across a majority of its surface area; or
wherein the bone interface layer (18) has a compressive modulus of at least 4 GPa across a majority of its surface area.

15. A method according to claim 14, wherein the implant (10) comprises a femoral knee implant, the bone interface layer (18) defining at least part of an anterior flange and at least one condyle, wherein the method further comprises:
forming the bone interface layer (18) such that the distance between a tip of the anterior flange and a tip of the condyle has a first value; and
cooling the over moulded implant body (12);
wherein the first value is selected such that contraction of the polymer material during cooling causes the anterior flange and the condyle to splay apart such that the distance between a tip of the anterior flange and a tip of the condyle has a second predetermined value.

## Patentansprüche

1. Chirurgisches Implantat (10), umfassend:
einen Implantatkörper (12), umfassend ein Polymermaterial, wobei der Implantatkörper (12) eine knochenzugewandte Oberfläche (14) aufweist; und
eine Knochengrenzschicht (18), die über mindestens einem Abschnitt der knochenzugewandten Oberfläche (14) bereitgestellt ist;
wobei die Knochengrenzschicht (18) aus einem Material ausgebildet ist, das eine höhere Steifigkeit als das Polymermaterial aufweist; und
wobei die Knochengrenzschicht (18) eine Polymersperreschicht (40) mit einer Dicke von mindestens 0,5 mm über einen Großteil des Oberflächenbereichs der Knochengrenzschicht (18) einschließt, wobei die Polymersperreschicht (40) für das Polymermaterial undurchlässig ist; **dadurch gekennzeichnet, dass** die Knochengrenzschicht (18) eine Biegesteifigkeit von mindestens 500 N/mm über einen Großteil ihres Oberflächenbereichs aufweist;
wobei die Knochengrenzschicht (18) einen Biegemodul von mindestens 1,0 GPa über einen Großteil ihres Oberflächenbereichs aufweist; oder
wobei die Knochengrenzschicht (18) einen Druckmodul von mindestens 4 GPa über einen Großteil ihres Oberflächenbereichs aufweist.

2. Implantat (10) nach Anspruch 1, wobei die Knochengrenzschicht (18) mindestens 1,7 mm dick über einen Großteil ihres Oberflächenbereichs ist; oder
wobei die Knochengrenzschicht (18) mindestens 15 % so dick wie die Dicke des darunterliegenden Implantatkörpers (12) entlang einer Achse senkrecht zu der Knochengrenzschicht (18) über einen Großteil ihres Oberflächenbereichs ist.

3. Implantat (10) nach einem der vorstehenden Ansprüche, wobei die Knochengrenzschicht (18) ferner eine knochenzugewandte erste poröse Schicht (41) umfasst.

4. Implantat (10) nach einem der vorstehenden Ansprüche, wobei die Knochengrenzschicht (18) ferner eine zweite poröse Schicht (42) umfasst, die dem Implantatkörper (12) zugewandt ist.

5. Implantat nach Anspruch 4, wobei die Polymersperreschicht Entlüftungslöcher (52) einschließt, die dimensioniert sind, um zu verhindern, dass das Polymermaterial hindurchtritt.

6. Implantat (10) nach Anspruch 4 oder 5, wobei die Polymersperreschicht (40) mindestens 25 % der Dicke der Knochengrenzschicht (18) über einen Großteil ihres Oberflächenbereichs beträgt.

7. Implantat (10) nach einem der Ansprüche 4 bis 6, wobei mindestens eine poröse Schicht (41, 42) ein perforiertes Blatt, ein Netz, eine 3D-gedruckte Schicht oder eine Schicht aus gesintertem Material umfasst.

8. Implantat (10) nach einem der vorstehenden Ansprüche, wobei die Knochengrenzschicht (18) aus Metall ausgebildet ist, optional wobei die Knochengrenzschicht (18) aus Titan ausgebildet ist.

9. Implantat (10) nach einem der Ansprüche 1 bis 7, wobei die Knochengrenzschicht (18) aus faserverstärktem Polyaryletherketone ausgebildet ist, optional wobei das Polyaryletherketone Polyetheretherketone, PEEK, ist.

10. Implantat (10) nach einem der vorstehenden Ansprüche, wobei das Polymermaterial des Implantatkörpers (12) ein Polyaryletherketone umfasst, optional wobei das Polyaryletherketone Polyetheretherketone, PEEK, ist.

11. Implantat (10) nach einem der vorstehenden Ansprüche, wobei die Knochengrenzschicht (18) über einer ausgewählten Region oder ausgewählten Regionen der knochenzugewandten Oberfläche (14) bereitgestellt ist; oder
wobei die Knochengrenzschicht (18) über im Wesentlichen der Gesamtheit der knochenzugewandten Oberfläche (14) bereitgestellt ist.

12. Implantat (10) nach einem der vorstehenden Ansprüche, wobei das Implantat (10) ein Knieimplantat umfasst.

13. Implantat (10) nach Anspruch 12, wobei das Knieimplantat ein femorales Knieimplantat umfasst, optional wobei sich die Knochengrenzschicht (18) auf mindestens einen Teil der knochenzugewandten Oberfläche eines anterioren Flansches und mindestens einen Kondylus des Implantatkörpers (12) erstreckt.

14. Verfahren zum Herstellen eines chirurgischen Implantats (10), das Verfahren umfassend:
Bereitstellen einer Knochengrenzschicht (18); und
Umspritzen eines Polymermaterials, um einen Implantatkörper (12) auszubilden, derart, dass die Knochengrenzschicht (18) über mindestens einem Abschnitt einer knochenzugewandten Oberfläche (14) des Implantatkörpers (12) bereitgestellt wird;
wobei die Knochengrenzschicht (18) aus einem Material ausgebildet ist, das eine höhere Steifigkeit als das Polymermaterial aufweist; und
wobei die Knochengrenzschicht (18) eine Polymersperreschicht (40) mit einer Dicke von mindestens 0,5 mm über einen Großteil des Oberflächenbereichs der Knochengrenzschicht (18) einschließt, wobei die Polymersperreschicht (40) für das Polymermaterial undurchlässig ist; und
wobei die Knochengrenzschicht (18) eine Biegesteifigkeit von mindestens 500 N/mm über einen Großteil ihres Oberflächenbereichs aufweist;
wobei die Knochengrenzschicht (18) einen Biegemodul von mindestens 1,0 GPa über einen Großteil ihres Oberflächenbereichs aufweist; oder
wobei die Knochengrenzschicht (18) einen Druckmodul von mindestens 4 GPa über einen Großteil ihres Oberflächenbereichs aufweist.

15. Verfahren nach Anspruch 14, wobei das Implantat (10) ein femorales Knieimplantat umfasst, wobei die Knochengrenzschicht (18) mindestens einen Teil eines anterioren Flansches und mindestens einen Kondylus definiert, wobei das Verfahren ferner umfasst:
Ausbilden der Knochengrenzschicht (18) derart, dass der Abstand zwischen einer Spitze des anterioren Flansches und einer Spitze des Kondylus einen ersten Wert aufweist; und
Kühlen des umspritzten Implantatkörpers (12);
wobei der erste Wert ausgewählt ist, derart, dass eine Kontraktion des Polymermaterials während des Abkühlens bewirkt, dass sich der anteriore Flansch und der Kondylus auseinanderspreizen, derart, dass der Abstand zwischen einer Spitze des anterioren Flansches und einer Spitze des Kondylus einen zweiten vorbestimmten Wert aufweist.

## Revendications

1. Implant chirurgical (10) comprenant :
un corps d'implant (12) comprenant un matériau polymère, ledit corps d'implant (12) ayant une surface orientée vers l'os (14) ; et
une couche d'interface osseuse (18) disposée sur au moins une partie de l'interface orientée vers l'os (14) ;
dans lequel la couche d'interface osseuse (18) est formée d'un matériau ayant une rigidité supérieure à celle du matériau polymère ; et
dans lequel la couche d'interface osseuse (18) comporte une couche barrière polymère (40) d'au moins 0,5 mm d'épaisseur sur une majorité de la surface de la couche d'interface osseuse (18), la couche barrière polymère (40) étant imperméable au matériau polymère ; **caractérisé en ce que** la couche d'interface osseuse (18) a une rigidité en flexion d'au moins 500 N/mm sur une majorité de sa surface ;
dans lequel la couche d'interface osseuse (18) a un module de flexion d'au moins 1,0 GPa sur une majorité de sa surface ; ou
dans lequel la couche d'interface osseuse (18) a un module de compression d'au moins 4 GPa sur une majorité de sa surface.

2. Implant (10) selon la revendication 1, dans lequel la couche d'interface osseuse (18) a une épaisseur d'au moins 1,7 mm sur une majorité de sa surface ; ou
dans lequel la couche d'interface osseuse (18) a au moins 15 % de l'épaisseur du corps d'implant sous-jacent (12) le long d'un axe perpendiculaire à la couche d'interface osseuse (18) sur une majorité de sa surface.

3. Implant (10) selon l'une quelconque des revendications précédentes, dans lequel la couche d'interface osseuse (18) comprend en outre une première couche poreuse orientée vers l'os (41).

4. Implant (10) selon l'une quelconque des revendications précédentes, dans lequel la couche d'interface osseuse (18) comprend en outre une seconde couche poreuse (42) orientée vers le corps d'implant (12).

5. Implant selon la revendication 4, dans lequel la couche barrière polymère comporte des trous d'aération (52) dimensionnés pour empêcher le matériau polymère de passer à travers.

6. Implant (10) selon la revendication 4 ou la revendication 5, dans lequel la couche barrière polymère (40) a au moins 25 % de l'épaisseur de la couche d'interface osseuse (18) sur une majorité de sa surface.

7. Implant (10) selon l'une quelconque des revendications 4 à 6, dans lequel au moins une couche poreuse (41, 42) comprend une feuille perforée, un maillage, une couche imprimée en 3D ou une couche de matériau fritté.

8. Implant (10) selon l'une quelconque des revendications précédentes, dans lequel la couche d'interface osseuse (18) est formée de métal, facultativement dans lequel la couche d'interface osseuse (18) est formée de titane.

9. Implant (10) selon l'une quelconque des revendications 1 à 7, dans lequel la couche d'interface osseuse (18) est formée de polyaryléthercétone renforcé par des fibres, facultativement dans lequel la polyaryléthercétone est la polyétheréthercétone, PEEK.

10. Implant (10) selon l'une quelconque des revendications précédentes, dans lequel le matériau polymère du corps d'implant (12) comprend une polyaryléthercétone, facultativement dans lequel la polyaryléthercétone est la polyétheréthercétone, PEEK.

11. Implant (10) selon l'une quelconque des revendications précédentes, dans lequel la couche d'interface osseuse (18) est disposée sur une région choisie ou des régions choisies de l'interface orientée vers l'os (14) ; ou
dans lequel la couche d'interface osseuse (18) est disposée sur sensiblement la totalité de la surface orientée vers l'os (14).

12. Implant (10) selon l'une quelconque des revendications précédentes, dans lequel l'implant (10) comprend un implant de genou.

13. Implant (10) selon la revendication 12, dans lequel l'implant de genou comprend un implant de genou fémoral, facultativement dans lequel la couche d'interface osseuse (18) s'étend sur au moins une partie de la surface orientée vers l'os d'une bride antérieure et sur au moins un condyle du corps d'implant (12).

14. Procédé de fabrication d'un implant chirurgical (10), le procédé comprenant :
la fourniture d'une couche d'interface osseuse (18) ; et
le surmoulage d'un matériau polymère pour former un corps d'implant (12) de telle sorte que la couche d'interface osseuse (18) est disposée sur au moins une partie d'une surface orientée vers l'os (14) du corps d'implant (12) ;
dans lequel la couche d'interface osseuse (18) est formée d'un matériau ayant une rigidité supérieure à celle du matériau polymère ; et
dans lequel la couche d'interface osseuse (18) comporte une couche barrière polymère (40) d'au moins 0,5 mm d'épaisseur sur une majorité de la surface de la couche d'interface osseuse (18), la couche barrière polymère (40) étant imperméable au matériau polymère ; et
dans lequel la couche d'interface osseuse (18) a une rigidité en flexion d'au moins 500 N/mm sur une majorité de sa surface ;
dans lequel la couche d'interface osseuse (18) a un module de flexion d'au moins 1,0 GPa sur une majorité de sa surface ; ou
dans lequel la couche d'interface osseuse (18) a un module de compression d'au moins 4 GPa sur une majorité de sa surface.

15. Procédé selon la revendication 14, dans lequel l'implant (10) comprend un implant de genou fémoral, la couche d'interface osseuse (18) définissant au moins une partie d'une bride antérieure et au moins un condyle, dans lequel le procédé comprend en outre :
la formation de la couche d'interface osseuse (18) de telle sorte que la distance entre une pointe de la bride antérieure et une pointe du condyle a une première valeur ; et
le refroidissement du corps d'implant surmoulé (12) ;
dans lequel la première valeur est sélectionnée de telle sorte que la contraction du matériau polymère pendant le refroidissement amène la bride antérieure et le condyle à s'écarter de telle sorte que la distance entre une pointe de la bride antérieure et une pointe du condyle a une seconde valeur prédéterminée.
